# EUROPEAN PATENT SPECIFICATION

(11) **EP 3 234 113 B1**
(45) Date of publication and mention of the grant of the patent: **23.01.2019**
(21) Application number: 15816413.7
(22) Date of filing: 14.12.2015
(51) Int. Cl.: C12N 7/00

(54) **A METHOD FOR A LARGE SCALE VIRUS PURIFICATION**
VERFAHREN ZUR VIRUSREINIGUNG IN GROSSEM UMFANG
PROCÉDÉ DE PURIFICATION DE VIRUS À GRANDE ÉCHELLE

(30) Priority: 16.12.2014 US 201462092413 P
(43) Date of publication of application: 25.10.2017
(73) Proprietor: GlaxoSmithKline Biologicals S.A., 1330 Rixensart (BE); The Chemo-Sero-Therapeutic Research Institute, Kumamoto-shi Kumamoto 860-8568 (JP)
(72) Inventor: GERKENS, Pascal, Charles, Louis, 1330 Rixensart (BE); LERUSE, Jean-Francois, Jose, Alain, Marie, Ghislain, 1330 Rixensart (BE)
(74) Representative: Furstoss, Olivia Aline
(86) International application number: PCT/EP2015/079534
(87) International publication number: WO 2016/096688

(56) References cited:
- WO-A1-2010/089339
- US-A- 4 118 477
- I. Toplin ET AL: "Large-Volume Purification of Tumor Viruses by Use of Zonal Centrifuges", Applied Microbiology, 1 May 1972 (1972-05-01), pages 1010-1014, XP055246292, UNITED STATES Retrieved from the Internet: URL:http://www.ncbi.nlm.nih.gov/pmc/articl es/PMC380491/pdf/applmicro00046-0194.pdf [retrieved on 2016-02-01]
- REIMER C B ET AL: "Purification of large quantities of influenza virus by density gradient centrifugation", JOURNAL OF VIROLOGY, THE AMERICAN SOCIETY FOR MICROBIOLOGY, US, vol. 1, no. 6, 1 December 1967 (1967-12-01), pages 1207-1216, XP002452565, ISSN: 0022-538X

## Description

### STATEMENT REGARDING FEDERALLY SPONSORED RESEARCH

This invention was made with United States government support under Contract # HHSO100200600011C awarded by the Department of Health and Human Services; the United States government has certain rights in the invention.

### TECHNICAL FIELD

The present invention relates to the field of virus purification.

### BACKGROUND

The development of cell culture-based technologies as an alternative to the traditional egg-based production systems for the manufacture of viral vaccines likely appears as the most rapid and most promising solution to overcome drawbacks and constraints associated with egg-based traditional systems. Commercial productions of viral vaccines typically require large quantities of virus as an antigen source. However, the egg-based process is vulnerable due to the varying biological quality of eggs and it lacks flexibility because of the logistic problems due to non-availability of large quantities of suitable eggs.

However, after production, whether produced on eggs or on cell culture, the produced virus requires to be recovered from the cell culture, and, when appropriate, to be purified. Methods for purifying viruses are known in the art. One typical method employs sucrose gradient centrifugation. For example, WO 2009/007608, WO 2008/135229 and WO 2010/089339 describe the purification of viruses by sucrose gradient centrifugations using a self-generating gradient.

Efficient vaccine production requires the growth of large scale quantities of virus produced in high yields from a host system. The cultivation conditions under which a virus is grown is of great significance with respect to achieving an acceptable high yield of the virus. Thus, in order to maximise the yield of the desired virus, both the system and the cultivation conditions must be adapted specifically to provide an environment which is advantageous for the production of the desired virus which is suitable for large scale production. One way is to improve the cell specific productivity, for example, by improving the culture medium, or increasing the cell density and/or reducing the virus material loss which occurs along the different purification steps. An additional level of complexity is introduced at the manufacturing level, when large-scale volumes or quantities need to be processed. When scaling-up, the risk of losing either in quality of the product (*e.g*. purity of the product) and/or in the yield of the product is significant. Such an occurrence requires further optimization of a process developed in a satisfactorily manner at small scale. Therefore, a need remains for providing methods to produce and to purify viruses with an adequate purity level and a good yield at large scale.

### SUMMARY OF THE INVENTION

In a first aspect of the present invention, there is provided a method for purifying a virus comprising at least the following steps:
a) obtaining a virus-containing fluid,
b) providing a pre-formed linear density gradient of a given range X%-Y% (v/w) (X being the lower limit and Y being the upper limit) in a centrifuge rotor having a capacity of at at least 8L,
c) adding the fluid to the pre-formed linear density gradient,
d) centrifuging so as to separate the virus from contaminating impurities, and
e) collecting the fractions comprising the purified virus,
wherein the linear part of the gradient encompasses the density percentage corresponding to the percentage at which the virus to be purified will migrate.

In a second aspect of the present invention, there is provided a method for the preparation of a vaccine comprising at least the step of purifying the virus to be used as an antigen in the vaccine according to the method of the invention and formulating said purified virus into a vaccine.

In a third aspect of the present invention, there is provided a method for preparing a vaccine comprising at least the following steps:
A) obtaining a virus-containing fluid,
B) providing a pre-formed linear density gradient of a given range X%-Y% (v/w) (X being the lower limit and Y being the upper limit) in a centrifuge rotor having a capacity of at least 4L, at least 6L, at least 8L, or at least 10L, wherein the linear part of the gradient encompasses the density percentage corresponding to the percentage at which the virus will migrate,
C) adding the fluid to the pre-formed linear density gradient,
D) centrifuging so as to separate the virus from contaminating impurities, and
E) collecting the fractions comprising the purified virus,
F) formulating the purified virus into a vaccine.

### DESCRIPTION OF DRAWINGS

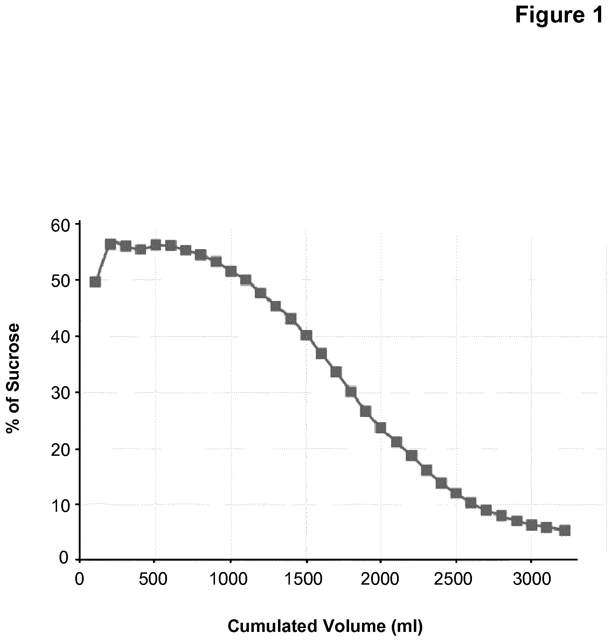

**Figure 1****:** Shape of a self-generating sucrose gradient obtained in a 3.2L rotor. Successive fractions of 100 ml of the formed gradient were collected and analyzed for their sucrose content by refractometry.

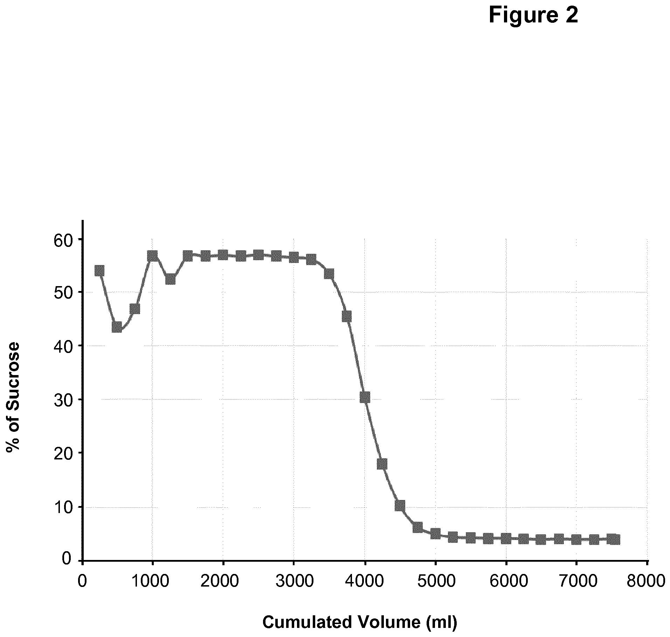

**Figure 2****:** Shape of a self-generating sucrose gradient obtained in a 8L rotor. Successive fractions of 250 ml of the formed gradient were collected and analyzed for their sucrose content by refractometry.

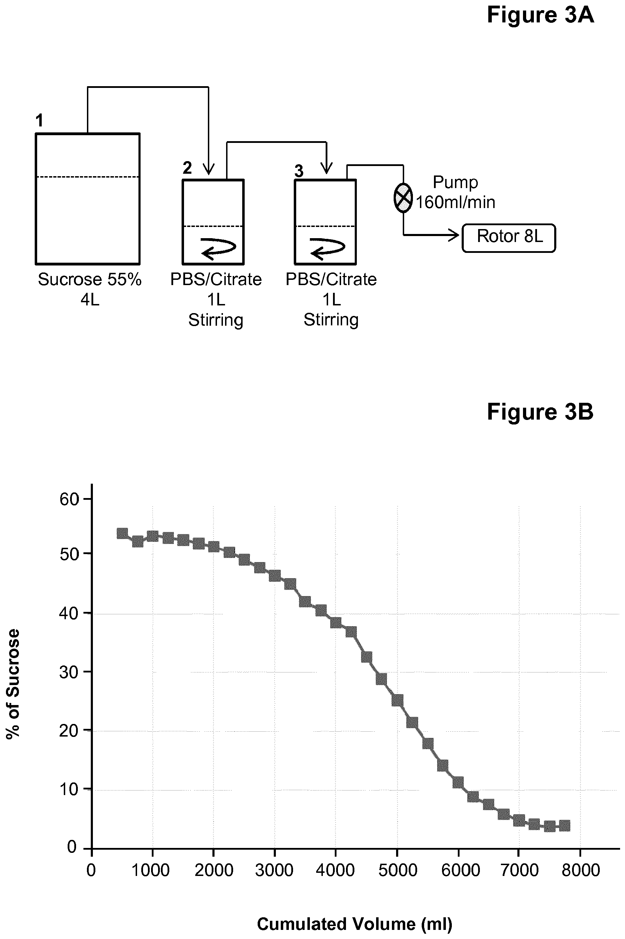

**Figure 3****:** Shape of a pre-formed sucrose gradient obtained in a 8L rotor. **Figure 3A** shows a diagram representing the method of formation of the pre-formed linear gradient - 3 containers comprising 4L of sucrose 55% (Container 1) and 1L of PBS/Citrate (Containers 2 and 3) are successively connected to one another, the third container being connected to a 8L rotor. **Figure 3B** shows the shape of the pre-formed sucrose obtained following the method provided in Figure 3A. Successive fractions of 250 ml of the formed gradient were collected and analyzed for their sucrose content by refractometry.

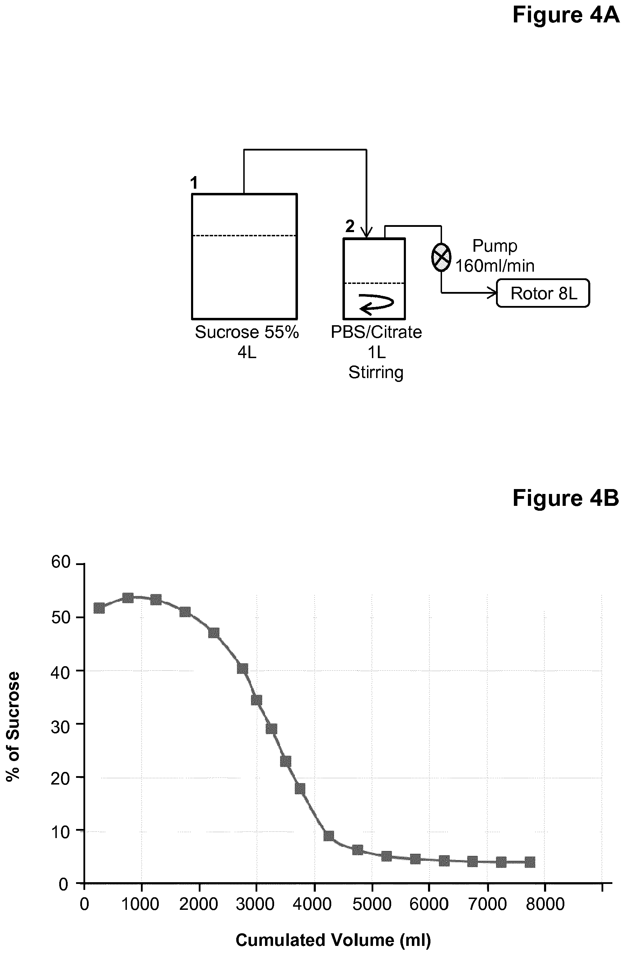

**Figure 4****:** Shape of a pre-formed sucrose gradient obtained in a 8L rotor. **Figure 4A** shows a diagram representing the method of formation of the pre-formed linear gradient - 2 containers comprising 4L of sucrose 55% (Container 1) and 1L of PBS/Citrate (Container 2) are successively connected to one another, the second container being connected to a 8L rotor. **Figure 3B** shows the shape of the pre-formed sucrose obtained following the method provided in Figure 3A. Successive fractions of 250 ml of the formed gradient were collected and analyzed for their sucrose content by refractometry.

### DETAILED DESCRIPTION

The present invention relates to an improved method for purifying viruses, whether produced in eggs or in cell culture, which is particularly useful for large scale production. A typical step used during virus purification is the step of density gradient ultracentrifugation, such as sucrose gradient for example.

Density gradient ultracentrifugation is a technique commonly used for purifying viruses, in particular enveloped viruses. It is especially used in vaccine manufacturing field. Typically, separation of particles, such as a virus and possible contaminating impurities, by density gradient ultracentrifugation may be rate-zonal, *i.e*. relies on particle size differences, or may be isopycnic, *i.e*. relies on particles density differences, or may rely on a combination of both. To achieve a rate-zonal separation, the density of the particles to be separated must be higher than the density of the gradient at any position of the gradient. In isopycnic methods, the density gradient encompasses the whole range of densities of the particles to be separated. Depending on their density and on the density gradient range, after centrifugation, particles either migrate to a position in the gradient where the density is equal to the particle density (particles are buoyant at that position and remain there), or the particles are pelleted at the bottom of the gradient. After centrifugation, only gradient fractions containing the virus will be collected. The pre-formed linear gradient of the invention may suitably be used for a rate-zonal separation or for an isopycnic separation. In one embodiment, the separation of virus from contaminating impurities in the method of purifying a virus according to the invention is isopycnic.

Typically, a density gradient may be linear or discontinuous. A linear gradient offers in particular the advantages of allowing a better separation between viral particles and residual contaminating impurities originating from the host used to produce the viral particles, and obtaining a purified virus which is concentrated in a small volume. When scaling up the process for producing a virus, and in particular when contemplating the use of a rotor with a high capacity in order to save the number of centrifuges required to process large volumes, the inventors observed that the known method they used for small rotors, consisting of creating a self-generating gradient from a single stock solution failed to provide a linear gradient. In order to obtain a linear gradient in rotors having a high capacity, the inventors observed that the gradient had to be pre-formed as linear, i.e. formed prior to starting the centrifugation.

By "pre-formed", it is meant in the sense of the present invention that the gradient is created, and thus formed in the rotor, prior to starting the centrifugation, as opposed to a self-generating gradient.

By "self-generating", it is meant in the sense of the present invention that the gradient is created, and thus formed, during centrifugation.

By "linear", it is meant in the sense of the present invention a density gradient in which a plot of concentration *versus* volume of the gradient yields a substantially straight line, as opposed to a "discontinuous", or step, gradient which is composed of layers, with abrupt changes in concentration from one layer to the next one.

By "plateau", it is meant in the sense of the present invention a significant gradient zone, *i.e.* a significant part of the gradient volume, over which the value of concentration, or percentage, remains approximately constant. For example, a plateau may represent 5%, 10%, 15%, or 20%, or more, of the total gradient volume. Plateaux may be present, in particular, at the ends of the gradient.

By "a value approximately constant", it is meant in the sense of the present invention in relation to a given value, values presenting a variation of 5% or less.

For clarity and simplicity purposes, the pre-formed linear gradient implemented in the method of the invention will be described below in relation to a sugar gradient. However, such a pre-formed linear gradient is not limited to sugar gradients. All the disclosure detailed thereafter is applicable to other types of gradient.

The inventors observed that if loading onto the rotor as many as 4, and up to 7, different layers of sugar with an increased density, they could obtain a gradient with an acceptable linearity after centrifugation. Alternatively, in order to avoid changing manually the sugar flask for each new sugar layer to be loaded, the inventors developed an automatic method of forming a pre-formed linear gradient at large scale based on progressive dilution.

By "dilution", it is meant that a gradient-forming stock solution at an appropriate percentage (w/v) is diluted in a single dilution solution, or in successive dilution solutions, prior to be injected into the rotor. As described in more detail below, the extent of dilution, the percentage of the stock solution, the volume of the stock solution, and the volume of the dilution solution(s) may vary depending, for instance, on the targeted range and/or the targeted steepness of the linear gradient to be pre-form.

By "progressive dilution", it is meant a drip system in which each drop of a concentrated solution is diluted in a dilution solution. For example, the concentrated solution may be the stock solution, or the diluted stock solution to be further diluted, when more than one dilution is contemplated as described below.

By "high capacity rotor", it is meant a rotor having a capacity of at least 4L, suitably at least 6L, suitably at least 8L, more suitably at least 10L, or higher.

By "rotor capacity", it is meant the maximum volume of liquid that a rotor can carry.

By "load capacity", it is meant the amount of virus-containing fluid litres which is loaded per litre of rotor.

The method according to the present invention is particularly advantageous when it is necessary to purify a virus produced at large scale. For example, the method according to the invention is suitably used when 1L, 50L, 100L, 200L, 500L, 1000L, or higher volumes of virus-containing fluids need to be processed and purified.

The method according to the present invention is applicable to the purification of any type of viruses and is irrespective of the types of host substrate used for producing viruses. For example, the method according to the invention is applicable to viruses produced on cell culture or on eggs.

The method of the invention is amenable to a wide range of viruses, any virus which is typically purified by density gradient ultracentrifugation. By way of examples, the method of the invention contemplates the purification of enveloped viruses, in particular, hepadnaviruses, herpes viruses, orthomyxoviruses, such as for example influenza virus, paramyxoviruses, such as measles virus, togaviruses, such as for example rubella virus, rhabdoviruses, such as for example rabies viruses, poxviruses, and retroviruses. In one embodiment, the viruses purified and produced by the method of the invention belong to the family of orthomyxoviruses, in particular, influenza virus.

The present invention does not rely on the use of a specific density gradient-type, and can, thus, be applicable to any density-type gradient. The choice of the density gradient medium is dependent on the virus which is to be purified and on the application which is intended for the resulting purified virus. For instance, enveloped viruses are less dense than non-enveloped viruses. This is known in the art. Specifically, depending on what purpose the virus produced according to the method is prepared for, a medium which does not affect virus integrity or its biological activity will be used. For example, when the virus prepared according to the method of the invention is intended for vaccination, the medium is chosen so as to maintain the immunogenicity of the virus, or of the viral antigen thereof. Sugar solutions, in particular sucrose, may be used to generate density gradients for use in the method according to the invention. Sucrose is particularly used to purify enveloped viruses, such as, but not limited to, influenza viruses. It is also a sugar frequently used in the field of vaccine manufacturing.

In one embodiment, the sugar used to create a pre-formed linear density gradient according to the method of the present invention is sucrose. This is advantageous for the purification of products for human use when safety considerations need to be kept in mind. However, the invention also contemplates the use of other sugars, including alcohol sugars, such as, for instance, sorbitol, or hydrogenated sugars, linear sugars, and modified sugars or any other sugar provided that the sugar has a solubility in water sufficient to produce solutions with densities specified according to the type of virus to be purified.

Pre-formed linear density gradients used in the method of the present invention are not limited to sugar gradients. The present invention also contemplates, as other illustrative examples, the use of gradients of salts, such as, for instance, caesium chloride, which is suitable for the purification of both enveloped viruses and non-enveloped viruses. Alternatively, the gradient may also be prepared with potassium tartrate, which presents the advantage of reaching a gradient with a higher density, compared to sucrose gradient. Accordingly, potassium tartrate gradients are, in particular, suitably employed for purifying non-enveloped viruses.

The pre-formed linear gradient to be provided in a rotor with a high capacity according to the method of the invention may be formed by progressive dilution of a stock solution in dilution solution(s) prior to injection into the rotor.

### Linearity

The linearity of the pre-formed gradient used in the method of the invention may vary in two ways. On the one hand, the slope which defines the steepness of the gradient may vary. The appropriate steepness may be chosen, for instance, depending on the desired level of virus purification and on the desired number of gradient fractions from which the purified virus is retrieved, i.e. on how concentrated should the virus fluid be after purification on a density gradient ultracentrifugation. Typically, the steeper the linear gradient is, the more concentrated the purified virus will be, as it will be present in a number of gradient fractions corresponding to a smaller volume. Conversely, the more gradual a linear gradient is, the less concentrated the purified virus will be, as it will be present in number of gradient fractions corresponding to a higher volume. Also, the steepness of the linear gradient will impact the capacity of separating the virus from the residual contaminating impurities. It is within the skilled person's abilities to determine the appropriate slope of the linear gradient, as needed, taking into account the above two elements, that is virus concentration and quality of separation of the virus from residual contaminating impurities.

On the other hand, the extent of linearity, *i.e*. the gradient volume over which said gradient is linear, may vary. The minimum requirement is that the pre-formed linear gradient used in the method of the invention is linear over the density range of the gradient encompassing the density percentage corresponding to the percentage at which the virus to be purified will migrate. Suitably, the pre-formed linear gradient used in the method of the invention is linear over at least 30%, suitably at least 50%, suitably at least 60%, suitably at least 70%, suitably at least 80%, suitably at least 90% of its total volume or the gradient is suitably linear over the entirety of its volume. In particular embodiments, the pre-formed linear density gradient used in the method of the invention is linear from over 50% to over 90%, from over 60% to over 80% of its total volume. In further particular embodiments, the pre-formed linear density gradient used in the method of the invention is linear over at least 70%. Accordingly, the pre-formed linear gradient to be used in the method of the invention may display plateaux at the ends of the gradient, whether at one end only, or at both ends. However, the added volume corresponding to the plateaux, if more than one plateau is present, suitably does not exceed 70%, suitably 50%, suitably 40%, suitably 30%, suitably 20%, suitably 10%, or 5% of the total volume of the gradient.

The linearity, as well as the extent of linearity, of a density gradient can be checked by using any known method in the art allowing to plot the percentage of the gradient *versus* the volume of the gradient. Linearity, as defined earlier, is established when such a plot yields a substantially straight line. Also, from such a plot can the slope be calculated, i.e. the steepness be monitored and adapted, if appropriate. For example, when sugar is the medium used for the density gradient, a refractometry analysis, such as a Brix analysis, which is well known in the art may be used to measure the sugar percentage (expressed in grams per 100 ml) in successive fractions of a given volume of the gradient.

### Gradient concentration range

The present invention is not limited to a particular percentage range of density gradients. Said range should be determined depending on the virus to be purified, in particular, depending on the virus density, on the densities of the expected residual contaminating impurities, and on the type of separation to be use, whether isopycnic or zonal. The skilled person will know how to associate with a given virus (having a given density) the appropriate percentage range of a density gradient, ensuring that the linear part of the pre-formed gradient encompasses the density percentage corresponding to the percentage at which the virus to be purified will migrate. The presence of a specific virus, or viral antigen thereof, at a certain range within a gradient can be monitored by standard techniques of protein detection, such as a Western-blot analysis using an antibody specific for the viral antigen. In the particular case of the influenza virus, the content of one of its surface antigen, the HA (Hemagglutinin) antigen, can be monitored by the SRID (Single Radial Immuno Diffusion) assay, which is a technique familiar to a person skilled in the art (J.M. Wood et al.: An improved single radial immunodiffusion technique for the assay of influenza haemagglutinin antigen: adaptation for potency determination of inactivated whole virus and subunit vaccines. J. Biol. Stand. 5 (1977) 237-247; J. M. Wood et al.: International collaborative study of single radial diffusion and immunoelectrophoresis techniques for the assay of haemagglutinin antigen of influenza virus. J. Biol. Stand. 9 (1981) 317-330). For a given desired percentage range, the skilled person will determine the appropriate dilution of a stock solution of an appropriate percentage (w/v) necessary in order to create a pre-formed linear gradient with an appropriate linearity to be used in the method of the present invention. A typical gradient range for purifying viruses is 0-55% (w/v). In one embodiment, the pre-formed linear density gradient to be used in the method according to the invention, such as a sucrose gradient, has a percentage range of 0-55% (w/v).

Stock solutions and dilution solution(s) may be prepared in water or added with salt at physiological concentrations (*e.g*. NaCl) and with a buffer (*e.g*. Tris or sodium phosphate). Stock solutions and dilution solutions may suitably be prepared in a buffered solution comprising salt at a physiological concentration, such as TBS or PBS. In one embodiment of the invention, stock solutions, such as for example sugar stock solutions, in particular sucrose, and dilution solutions are prepared in a citrate-containing Phosphate Buffer Saline (PBS) solution, as this type of solution advantageously prevents virus aggregation during the centrifugation.

### Stock solution

By "stock solution", it is meant the gradient-forming solution which will be diluted in order to provide a pre-formed linear density gradient in a high capacity rotor according to the method of the invention. The gradient-forming stock solution to be used in order to prepare a pre-formed linear gradient of a given range X%-Y% (w/v) (X being the lower limit and Y being the upper limit), such as a sugar gradient, is suitably of a percentage which equals Y, or is higher. A non-limiting typical sugar gradient range for purifying viruses is 0-55% (w/v). For example, if a sugar gradient of 0-55% (w/v) is targeted, the stock solution of sugar may suitably be a 55% (w/v) sugar solution, or higher.

The volume of the stock solution is selected so as to obtain a pre-formed linear gradient reaching the upper limit of the expected range. For a given range X%-Y% (X being the lower limit and Y being the upper limit) (w/v), the volume of the stock solution is selected so as to obtain a pre-formed linear gradient reaching the upper limit Y% of the range. The inventors observed that the ratio "volume of the stock solution/volume of the dilution solution" is a factor to be taken into account. The stock solution suitably has a higher volume than the dilution solution. For example, such a ratio is suitably 2:1, 3:1, 4:1, 5:1 or 6:1.

The volume of the stock solution is also selected in accordance with the capacity of the rotor. Suitably, the volume of the stock solution does not exceed the capacity of the rotor. In embodiments, wherein the centrifugation is performed in batch, the volume of the stock solution matches the rotor capacity. When the centrifugation is performed in a continuous mode, the volume of the stock solution may have a volume of 90% of the rotor capacity, or less, suitably 80% or less. In embodiments, wherein the centrifugation is performed in a continuous mode, the volume of the gradient-forming stock solution ranges from 40% to 80% of the rotor capacity, from 50% to 60% of the rotor capacity or is 50% of the rotor capacity. In particular embodiments, wherein the rotor is a 8L rotor, the gradient-forming stock solution has a volume ranging from 3.2L to 6.4L, or from 4L to 4.8L. In a further particular embodiment, wherein the rotor is a 8L rotor, the gradient-forming stock solution has a volume of 4L.

### Dilution solutions

The dilution solution may comprise no sugar at all. Alternatively, it may also comprise some sugar, typically at a percentage lower than the percentage of the stock solution. Typically, a stock solution of a given percentage is diluted in dilution solution(s) prior to being injected into a rotor of a given capacity. The steepness of the pre-formed linear gradient to be used in the method according to the invention can be adjusted by adapting the dilution rate. Typically, the steeper a gradient is, the more likely it is to observe the presence of plateaux at the end(s) of the gradient.

Diminishing the dilution rate helps reduce the plateaux at the end(s) of the gradient, increase the extent of linearity, *i.e*. increase the volume of the gradient over which the gradient is linear, and make the linear gradient more gradual, *i.e*. with a softer slope. Diminishing the dilution rate may be achieved for example, by adding sugar or increasing its percentage in the dilution solution. An alternative option to diminish the dilution rate is to proceed to a multiple dilution.

By "multiple dilution", it is meant successive dilutions. For example, the gradient-forming stock solution may be progressively diluted twice or more, *i.e*. diluted in a first dilution solution, which diluted stock solution is in turn progressively diluted in a second dilution solution, and so on before being injected into the rotor. In one embodiment, the pre-formed linear gradient is formed by progressively diluting a given stock solution twice, *i.e*. diluted in a first given dilution solution, which diluted stock solution is progressively diluted in a second given dilution solution prior to being injected into the rotor. In particular embodiments where such multiple dilution is performed, the dilution solutions do not comprise any sugar at all and possibly have the same volume.

The volume of the dilution solutions used to create a pre-formed linear gradient to be used in the method according to the invention may vary. In particular, the volume depends on the volume of the gradient-forming stock solution, and on the targeted dilution rate, *i.e*. on the expected steepness of the pre-formed linear gradient. For example, a given dilution solution may suitably have a volume of at least 10%, at least 15%, at least 20%, at least 25%, at least 30%, or at least 50% of the stock solution.

In one embodiment, for an expected gradient of a range X%-Y% (w/v) (X being the lower limit and Y being the upper limit), a stock solution having a percentage Y% is first progressively diluted in a dilution solution having a volume of 25% of the volume of the stock solution, which diluted stock solution is progressively diluted in a second dilution solution having a volume of 25% of the volume of the stock solution.

Typically the different containers each comprising the gradient-forming stock solution and the dilution solution(s), and the rotor which will accommodate the pre-formed linear gradient are successively connected with one another, the last container being connected to the rotor by means of a pump. Said pump, once turned on, creates vacuum resulting in the aspiration of the stock solution into the first dilution solution, which is in turn aspirated, and thus progressively diluted, in the next dilution solution, and so on, depending on how many dilution solutions are used, before the final diluted solution is injected into the rotor. The pump debit is chosen in an appropriate range so as to not disturb the gradient obtained and formed by progressive dilution. One non-limiting example of pump debit to be suitably used in the method according to the invention ranges from 100 to 200 ml/min, suitably is 160 ml/min.

### Centrifugation

Centrifugation conditions are standard conditions. It is within the skilled person's abilities to determine the appropriate conditions, such as for example the centrifugal force, the operation time, the flow rate speed, or the banding time, if appropriate. The determination of said conditions shall take into account the type of virus-containing fluid to be purified, the type of virus, the type of contaminating impurities, the mode of centrifugation, whether in batch or in continuous, the type of separation, whether zonal or isopycnic. In particular, information provided in manufacturer's instructions as how to use rotors and centrifuges may guide the skilled person in the selection of appropriate centrifugation conditions. As described earlier, the skilled person may use any standard techniques of protein detection, such as a Western-blot analysis using an antibody specific for a viral antigen, or in the particular case of the influenza virus, the SRID assay in order to detect the presence of the virus and/or monitor whether acceptable virus yield and/or virus purity after the centrifugation on a pre-formed linear density gradient of the invention are obtained.

Centrifugation may be performed in a continuous mode, in a semi-continuous mode or in successive batches. In one embodiment, the centrifugation during the method of purifying a virus according to the invention is performed in a continuous mode. Centrifugation in a continuous mode is advantageously used at large scale, when large volumes of virus-containing fluids needs to be processed and purified.

Depending on whether the centrifugation is performed in a batch mode or in a continuous mode, the loading of the pre-formed linear gradient may vary. In batch mode, the pre-formed linear gradient may be loaded in an empty rotor at rest. In such embodiments, the loaded volume may match the rotor capacity. Alternatively, when the centrifugation is performed in a continuous mode, the rotor may be pre-filled with an appropriate buffer solution at rest, and while injecting the pre-formed linear gradient into the rotor still at rest, said buffer solution is progressively replaced with the pre-formed linear gradient. Such a pre-fill presents the advantage of avoiding the formation of bubbles. In some embodiments, wherein the centrifugation is performed in a continuous mode, the volume of the pre-formed linear gradient which is injected into the rotor corresponds to the volume of the gradient-forming stock solution, *i.e*. the injection is stopped when the container comprising the stock solution is empty.

Once the pre-formed linear gradient is loaded into the rotor, the virus-containing fluid may be loaded onto the gradient at rest, and the centrifugation is started after the virus-containing has been loaded onto the gradient. Such embodiments are suitably carried out when the centrifugation is performed in batch. Alternatively, the centrifugation may be started after the pre-formed linear gradient is loaded into the rotor, and once the appropriate centrifugal force is reached, the virus-containing fluid maybe loaded onto the gradient. Such alternate embodiments are suitably implemented when the centrifugation is performed in a continuous mode.

The appropriate centrifugal force may be chosen by the skilled person and may depend on the type of rotor and centrifuge used. As non-limiting examples, a centrifugal force ranging from at least 20,000 g to 200,000 g, suitably ranging from 90,000 g to 150,000 g, suitably ranging from 100,000 g to 120,000 g, is applied to the rotor in the centrifuge. Once all the virus-containing fluid has been processed, and an adequate operation time allowed, the centrifugation is stopped and the gradient fractions containing the virus are collected.

### Virus-containing fluid

The virus-containing fluid to be purified in the sense of the invention is not limited to crude fluids, but also contemplates fluids which comprise partially purified viruses. For instance, purification may include a number of different filtration, concentration and/or other separation steps such as ultrafiltration, centrifugation, chromatography (such as ion exchange chromatography) and adsorption steps in a variety of combinations. A clarification step may be required in order to separate the virus from cellular material contaminant, in particular, floating cells or cell debris, or from eggs-derived contaminants originating from allantoic fluids.

The term "crude" in the sense of the present invention means that no purification has been performed on the virus-containing fluid after its harvest, and, thus, may contain any kind of contaminants to varying extents. As a non-limiting example, when the virus has been produced by inoculating cells with said virus and when, after infection, newly formed viruses are released to the cell culture medium or supernatant, then said culture medium, which comprises the virus, designates an example of a crude fluid. Another example of a crude fluid which may be cited is the allantoic fluid harvested after inoculation of virus onto embryonated eggs and virus cultivation. Accordingly, the terms "partially purified" encompass any intermediate purification status, *i.e*. a fluid which has been the subject of any purification step, for instance, any of the steps mentioned above, individually, or in any combination.

In one embodiment, the virus-containing fluid according to the invention is the culture medium collected after infection of the cells with the virus of interest, such as influenza virus for example, and its release into the culture medium. In another embodiment, the virus-containing fluid according to the invention is the allantoic fluid collected after inoculation of eggs with a virus of interest, such as influenza virus.

In another suitable embodiment, the virus-containing fluid according to the invention has been clarified before being added to a pre-formed linear density gradient according to the invention. This clarification may be done by filtration. Alternatively, centrifugation and/or filtration may be combined together, in any order, for achieving the desired clarification level of the virus preparation. Although not required, a multiple filtration process may be carried out, like a two- or three-stage process consisting, for instance, in sequentially and progressively removing impurities according to their size, using filters with appropriate nominal pore size, in particular, filters with decreasing nominal pore size, allowing to start removing large precipitates and cell debris. In addition, single stage operations employing a relatively tight filter or centrifugation may also be used for clarification. More generally, any clarification approach including, but not limited to, dead-end filtration, depth filtration, microfiltration, or centrifugation, which provide a filtrate of suitable clarity to not foul the membrane and/or resins in subsequent steps, will be acceptable to use in the clarification step of the present invention.

Although it is not required, it may be suitable to concentrate the virus-containing fluid prior to loading it on the pre-formed linear density gradient according to the invention, in order to reduce the fluid volume to be loaded. Accordingly, the present invention also contemplates a virus-containing fluid which has been concentrated, prior to loading on the density gradient. Therefore, the virus-containing fluid may be subjected to ultrafiltration (sometimes referred to as diafiltration when used for buffer exchange), for instance on an 750 kD membrane, for concentrating the virus and/or buffer exchange. This step is particularly advantageous when the virus to be purified is diluted, as is the case when pooling viral harvest collected by perfusion over a few days post-inoculation. The process used to concentrate the virus can include any filtration process where the concentration of virus is increased by forcing diluent to be passed through a filter in such a manner that the diluent is removed from the virus suspension whereas the virus is unable to pass through the filter and thereby remains in concentrated form in the virus preparation.

Ultrafiltration may comprise diafiltration which is an ideal way for removal and exchange of salts, sugars, non-aqueous solvents, removal of material of low molecular weight, of rapid change of ionic and/or pH environments. Microsolutes are removed most efficiently by adding solvent to the solution being ultrafiltered at a rate equal to the ultrafiltration rate. This washes microspecies from the solution at a constant volume, isolating the retained virus. Diafiltration is particularly advantageous when a downstream step requires that a specific buffer be used in order to get an optimal reaction. Concentration and diafiltration may be implemented at any suitable step of the purification process, when it is wanted to remove undesirable compounds, such as sucrose, after a sucrose gradient ultracentrifugation, or such as formaldehyde, after a step of virus inactivation with formaldehyde. The system is composed of three distinct process streams: the feed solution (comprising the virus), the permeate and the retentate. Depending on the application, filters with different pore sizes may be used. The filter composition may be, but is not limited to, regenerated cellulose, polyethersulfone, polysulfone, or derivatives thereof. The membranes can be flat sheets (also called falt screens) or hollow fibers.

In one embodiment, the virus-containing fluid has been concentrated by ultrafiltration/diafiltration prior to loading it on the pre-formed linear density gradient used in the method of the present invention.

The purification method according to the present invention may include additional steps, in addition to the step of density gradient ultracentrifugation as herein claimed. These steps may be implemented before or after proceeding to said density gradient ultracentrifugation step. Specifically, the virus preparation obtained after using the density gradient ultracentrifugation step according to the present invention may be further purified, by implementing any one of the previously cited virus purification techniques, such as filtration, ultracentrifugation (including gradient ultracentrifugation), chromatography (such as ion exchange chromatography) and adsorption steps in a variety of combinations.

In one embodiment, the method of the present invention further comprises at least one step selected from the group of: filtration, ultrafiltration/diafiltration, ultracentrifugation and chromatography, or any combination thereof. Depending on the purity level that is desired, the above steps may be combined in any way.

Alternatively, it is also possible to further purify viruses by chromatography, including ion exchange, anionic or cationic, chromatography, size exclusion, such as gel filtration or gel permeation, chromatography, hydrophobic interaction chromatography, hydroxyapatite or any combination thereof. As mentioned above, the chromatography steps may be implemented in combination with other purifications steps, such as density gradient ultracentrifugation.

### Eggs

Virus-containing fluids, such as influenza virus, according to the invention may be derived from the conventional embryonated egg method, by growing influenza virus in eggs and purifying the harvested allantoic fluid.

### Cells

The virus to be purified according to the method of the invention may be produced on cell culture. The method according to the present invention is applicable to any type of cells, whether adherent cells grown on micro-carriers or suspension cells.

Cells may be grown in various ways, such as for example, using batch, fed-batch, or continuous systems, such as perfusion systems. Perfusion is particularly advantageous when high cell density is desired. High cell density may be particularly advantageous in order to maximise the amount of virus which can be produced from a given cell type.

The cells used to produce a virus-containing fluid to be purified according to the method of the invention can in principle be any desired type of animal cells which can be cultured in cell culture and which can support virus replication. They can be either primary cells or continuous cell lines. Genetically stable cell lines are preferred. Mammalian cells are particularly suitable, for example, human, hamster, cattle, monkey or dog cells. Alternatively, insect cells are also suitable, such as, for instance, SF9 cells or Hi-5 cells

A number of mammalian cell lines are known in the art and include PER.C6, HEK cells, human embryonic kidney cells (293 cells), HeLa cells, CHO cells, Vero cells and MDCK cells.

Suitable monkey cells are, for example, African green monkey cells, such as kidney cells as in the Vero cell line. Suitable dog cells are, for example, kidney cells as in the MDCK cell line.

Suitable mammalian cell lines for growing influenza virus include MDCK cells, Vero cells, or PER.C6 cells. These cell lines are all widely available, for instance, from the American Type Cell Culture (ATCC) collection.

Alternatively, cell lines for use in the invention may be derived from avian sources, such as chicken, duck, goose, quail or pheasant. Avian cell lines may be derived from a variety of developmental stages including embryonic, chick and adult. In particular, cell lines may be derived from the embryonic cells, such as embryonic fibroblasts, germ cells, or individual organs, including neuronal, brain, retina, kidney, liver, heart, muscle, or extraembryonic tissues and membranes protecting the embryo. Chicken embryo fibroblasts (CEF) may be used. Examples of avian cell lines include avian embryonic stem cells (WO01/85938) and duck retina cells (WO05/042728). In particular, the EB66® cell line derived from duck embryonic stem cells is contemplated in the present invention. Other suitable avian embryonic stem cells include the EBx® cell line derived from chicken embryonic stem cells, EB45, EB14 and EB14-074 (WO2006/108846). This EBx cell line presents the advantage of being a stable cell line whose establishment has been produced naturally and did not require any genetic, chemical or viral modification. These avian cells are particularly suitable for growing influenza viruses.

According to a particular embodiment, the method of the invention, when the virus to be purified is produced on cell culture, uses EB66® cells.

Cell culture conditions (temperature, cell density, pH value, etc ...) are variable over a very wide range owing to the suitability of the cells employed and can be adapted to the requirements of particular virus growth conditions details. It is within the skilled in the art person's capabilities to determine the appropriate culture conditions, as cell culture is extensively documented in the art (see, for example, Tissue Culture, Academic Press, Kruse and Paterson, editors (1973), and R.I. Freshney, Culture of animal cells: A manual of basic technique, fourth edition (Wiley-Liss Inc., 2000, ISBN 0-471-34889-9).

Before harvesting, cell infection may last for 2 to 10 days. The optimal time to harvest the cell-produced virus is usually based on the determination of the infection peak. For example, the CPE (CytoPathic Effect) is measured by monitoring the morphological changes occurring in host cells after virus inoculation, including cell rounding, disorientation, swelling or shrinking, death, detachment from the surface. The detection of a specific viral antigen may also be monitored by standard techniques of protein detection, such as a Western-blot analysis. Harvest can then be collected when the desired detection level is achieved. In the particular case of influenza virus, the content of HA may be monitored any time post-inoculation of the cells with the virus, by the SRD assay (Wood, JM, et al. (1977). J. Biol. Standard. 5, 237-247), which is a technique familiar to a person skilled in the art. Additionally, the SRD assay may also be used for determining the optimal cell density range required to obtain an optimized virus yield.

### Immunogenic compositions

At the end of the purification, the virus preparation obtained according to the method of the present invention may be suitably subject to sterile filtration, as is common in processes for pharmaceutical grade materials, such as immunogenic compositions or vaccines, and known to the person skilled in the art. Such sterile filtration can for instance suitably be performed by filtering the preparation through a 0.22 µm filter. After sterile preparation, the virus or viral antigens are ready for clinical use, if desired. The virus purified according to the method of the invention may suitably be formulated to be included in an immunogenic composition, such as in a vaccine. Accordingly, a method for the preparation of a vaccine, such as an influenza virus vaccine, comprising at least the step of purifying a virus to be used as an antigen in the vaccine according to the method of the invention and formulating said purified virus into a vaccine is also contemplated in the present invention.

The immunogenic compositions, in particular vaccines, may generally be formulated in a sub-virion form, *e.g*. in the form of a split virus, where the lipid envelope has been dissolved or disrupted, or in the form of one or more purified viral proteins (subunit vaccine). As an alternative, the immunogenic compositions may include a whole virus, *e.g*. a live attenuated whole virus, or an inactivated whole virus.

Methods of splitting viruses, such as influenza viruses, are well known in the art (WO02/28422). Splitting of the virus is carried out by disrupting or fragmenting whole virus whether infectious (wild-type or attenuated) or non-infectious (inactivated) with a disrupting concentration of a splitting agent. Splitting agents generally include agents capable of breaking up and dissolving lipid membranes. Traditionally, split influenza virus was produced using a solvent/detergent treatment, such as tri-n-butyl phosphate, or diethylether in combination with Tween™ (known as "Tween-ether" splitting) and this process is still used in some production facilities. Other splitting agents now employed include detergents or proteolytic enzymes or bile salts, for example sodium deoxycholate. Detergents that can be used as splitting agents include cationic detergents e.g. cetyl thrimethyl ammonium bromide (CTAB), other ionic detergents, *e.g*. sodium lauryl sulphate (SLS), taurodeoxycholate, or non-ionic detergents such as Tween or Triton X-100, or combination of any two or more detergents.

The splitting process may be carried out as a batch, continuous or semi-continuous process. When implemented in batch, the split virus may require an additional step of purification, such as a chromatography step. It is not necessary to implement a splitting step as such, as it is possible to perform the splitting simultaneously to a purification step. For instance, a detergent may be added to the pre-formed linear gradient aimed at purifying viral proteins by ultracentrifugation, as described above. In one embodiment, the method according to the invention further comprises a splitting step performed in batch with a detergent, in particular, Triton X-100.

Alternatively, splitting may occur before or after the density gradient centrifugation step as claimed herein.

For the safety of vaccines, it may be necessary to reduce infectivity of the virus suspension along different steps of the purification process. The infectivity of a virus is determined by its capacity to replicate on a cell line. Therefore, the method according to the present invention, optionally, includes at least one virus inactivation step. The inactivation may be performed by using, for instance, beta-propiolactone (BPL), formaldehyde, or UV, or any combination thereof, which can take place either before or after the density gradient centrifugation step of the invention. In one embodiment, the method according to the invention further comprises an inactivation step, performed with BPL for example, which step suitably taking place after the density gradient ultracentrifugation of the invention. The conditions of viral inactivation may vary and will be determined, in particular, by assessing the residual virus infectivity by measuring the Tissue Culture Infectious dose (TCID₅₀/ml).

Immunogenic compositions of the present invention, including vaccines, can optionally contain the additives customary for vaccines, in particular substances which increase the immune response elicited in a patient who receives the composition, *i.e*. so-called adjuvants.

In one embodiment, immunogenic compositions are contemplated, which comprise a virus or viral antigen of the present invention admixed with a suitable pharmaceutical carrier. In a specific embodiment, they comprise an adjuvant.

### Influenza virus

Viruses or viral antigens may be derived from an Orthomyxovirus, such as influenza virus. Orthomyxovirus antigens may be selected from one or more of the viral proteins, including hemagglutinin (HA), neuraminidase (NA), nucleoprotein (NP), matrix protein (M1), membrane protein (M2), one or more of the transcriptase (PB1, PB2 and PA). Particularly suitable antigens include HA and NA, the two surface glycoproteins which determine the antigenic specificity of the Influenza subtypes.

The influenza virus can be selected from the group of human influenza virus, avian influenza virus, equine influenza virus, swine influenza virus, feline influenza virus. Influenza virus is more particularly selected in strains A, B and C, preferably from strains A and B.

Influenza antigens may be derived from interpandemic (annual or seasonal) influenza strains. Alternatively, influenza antigens may be derived from strains with the potential to cause a pandemic outbreak (*i.e*., influenza strains with new hemagglutinin compared to hemagglutinin in currently circulating strains, or influenza strains which are pathogenic in avian subjects and have the potential to be transmitted horizontally in the human population, or influenza strains which are pathogenic to humans). Depending on the particular season and on the nature of the antigen included in the vaccine, the influenza antigens may be derived from one or more of the following hemagglutinin subtypes: H1, H2, H3, H4, H5, H6, H7, H8, H9, H10, H11, H12, H13, H14, H15 or H16. Preferably, the influenza virus or antigens thereof are from H1, H2, H3, H5, H7 or H9 subtypes. In one embodiment, the influenza viruses are from H2, H5, H6, H7 or H9 subtypes. In an alternative embodiment, the influenza viruses are from H1, H3 or B subtypes.

### Influenza virus immunogenic compositions

The method of purification according to the invention is particularly suitable for preparing influenza virus immunogenic compositions, including vaccines. Various forms of influenza virus are currently available. They are generally based either on live virus or inactivated virus. Inactivated vaccines may be based on whole virions, spilt virions or purified surface antigens (including HA). Influenza antigens can also be presented in the form of virosomes (nucleic acid-free viral-like liposomal particles).

Virus inactivation methods and splitting methods have been described above and are applicable to influenza virus.

Influenza virus strains for use in vaccines change from season to season. In the current interpandemic period, vaccines typically include two influenza A strains and one influenza B strain. Trivalent vaccines are typical, but higher valence, such as quadrivalence, is also contemplated in the present invention. The invention may also use HA from pandemic strains (*i.e*. strains to which the vaccine recipient and the general human population are immunologically naive), and influenza vaccines for pandemic strains may be monovalent or may be based on a normal trivalent vaccine supplemented by a pandemic strain.

Compositions of the invention may include antigen(s) from one or more influenza virus strains, including influenza A virus and/or influenza B virus. In particular, a trivalent vaccine including antigens from two influenza A virus strains and one influenza B virus strain is contemplated by the present invention. Alternatively a quadrivalent vaccine including antigens from two influenza A virus strains and two influenza B virus strains is also within the scope of the present invention.

The compositions of the invention are not restricted to monovalent compositions, *i.e*. including only one strain type, *i.e*. only seasonal strains or only pandemic strains. The invention also encompasses multivalent compositions comprising a combination of seasonal strains and/or of pandemic strains. In particular, a quadrivalent composition, which may be adjuvanted, comprising three seasonal strains and one pandemic strain falls within the scope of the invention. Other compositions falling within the scope of the invention are a trivalent composition comprising two A strains and one B strain, such as H1N1, H3N2 and B strains, and a quadrivalent composition comprising two A strains and two B strains of a different lineage, such as H1N1, H3N2, B/Victoria and B/Yamagata.

HA is the main immunogen in current inactivated influenza vaccines, and vaccine doses are standardized by reference to HA levels, typically measured by SRD. Existing vaccines typically contain about 15 µg of HA per strain, although lower doses can be used, *e.g*. for children, or in pandemic situations, or when using an adjuvant. Fractional doses such as a half (*i.e*. 7.5 µg HA per strain) or a quarter have been used, as have higher doses, in particular, 3x or 9x doses. Thus immunogenic compositions of the present invention may include between 0.1 and 150 µg of HA per influenza strain, particularly, between 0.1 and 50 µg, *e.g*. 0.1-20 µg, 0.1-15 µg, 0.1-10 µg, 0.1-7.5 µg, 0.5-5 µg, etc. Particular doses include about 15, about 10, about 7.5, about 5 µg per strain, about 3.8 µg per strain and about 1.9 µg per strain.

Once an influenza virus has been purified for a particular strain, it may be combined with viruses from other strains to make a trivalent vaccine, for example, as described above. It is more suitable to treat each strain separately and to mix monovalent bulks to give a final multivalent mixture, rather than to mix viruses and degrade DNA and purify it from a multivalent mixture.

Adjuvant compositions may comprise an oil-in-water emulsion which comprise a metabolisable oil and an emulsifying agent. In order for any oil in water composition to be suitable for human administration, the oil phase of the emulsion system has to comprise a metabolisable oil. The meaning of the term metabolisable oil is well known in the art. Metabolisable can be defined as 'being capable of being transformed by metabolism' (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts, seeds, and grains are common sources of vegetable oils. Synthetic oils are also part of this invention and can include commercially available oils such as NEOBEE® and others.

The oil-in-water emulsion further comprises an emulsifying agent. The emulsifying agent may suitably be polyoxyethylene sorbitan monooleate. Further, said emulsifying agent is suitably present in the vaccine or immunogenic composition 0.125 to 4% (v/v) of the total volume of the composition.

The oil-in-water emulsion optionally comprises a tocol. Tocols are well known in the art and are described in EP0382271. A suitable tocol is alpha-tocopherol or a derivative thereof such as alpha-tocopherol succinate (also known as vitamin E succinate). Said tocol is suitably present in the adjuvant composition in an amount 0.25% to 10% (v/v) of the total volume of the immunogenic composition.

The invention will be further described by reference to the following, non-limiting, examples.

### Example I: Gradient formation for a 3.2L rotor - Self generating gradient

The continuous flow ultracentrifuge was a KII centrifuge using a K3 rotor (3.2L capacity) from Alfa Wasserman. The K3/3.2L rotor was filled up with a buffer solution of PBS/Citrate 125mM. Once filled up, the centrifuge was started while keeping circulating the buffer in closed circuit to drive all air out of the rotor. The centrifuge was then stopped and one half of the volume (1.6L) of the buffer solution PBS/Citrate 125 mM was replaced with a solution of PBS/Citrate 125mM containing 55% sucrose. After replacement, the rotor was first slowly accelerated to 4000 rpm, and then further to a final speed of 35000 rpm. As the rotor was gradually accelerated to 4000 rpm, a linear gradient from 0% to 55% sucrose was formed within the rotor. After formation, successive fractions of 100 ml of the gradient were collected and analyzed for their sucrose content by refractometry (% Brix) in order to check the linearity of the gradient. Results are shown in Figure 1.

### Results - Conclusions

The obtained gradient was substantially linear, *i.e*. linear over most of its volume.

### Example II: Gradient formation for a 8L rotor - Self generating gradient

The same method as described in Example I was applied for the formation of a gradient in a 8L rotor. The continuous flow ultracentrifuge was a KII centrifuge using a K10 rotor (8L capacity) from Alfa Wasserman. The K10/8L rotor was filled up with a buffer solution of PBS/Citrate 125mM. Once filled up, the centrifuge was started while keeping circulating the buffer in closed circuit to drive all air out of the rotor. The centrifuge was then stopped and one half of the volume (4L) of the buffer solution PBS/Citrate 125 mM was replaced with a solution of PBS/Citrate 125mM containing 55% sucrose. After replacement, the rotor was first slowly accelerated to 4000 rpm, and then further to a final speed of 35000 rpm. After formation, successive fractions of 250 ml of the gradient were collected and analyzed for their sucrose content by refractometry (% Brix) in order to check the linearity of the gradient. Results are shown in Figure 2.

### Results - Conclusions

No linear gradient was obtained. The resulting gradient was a step gradient - A first plateau around 55% sucrose was observed over the first 3.5L of the gradient and a second plateau was observed around 5% sucrose over the last 3L of the gradient. The linear part of the gradient only extended from 3.5L to 5L, i.e. the gradient was linear over 1.5L, that is over only about 20% of its total volume.

### Example III: Gradient formation for a 8L rotor - Pre-formed gradient

**III.1** The same centrifuge and rotor as in Example II were used. 3 containers were used. The first one (container 1) contained 4L of PBS/Citrate 125mM buffer comprising 55% sucrose. The second one (container 2) and the third one (container 3) each contained 1L of PBS/Citrate 125mM buffer. The container 1 was connected to the container 2 that was connected to the container 3. Liquids in the containers 2 and 3 were mixed by a magnetic stirrer at a speed of 90 rpm. The container 3 was connected through a pump to the bottom part of the K10/8L rotor that was filled up with PBS/Citrate 125mM buffer. When the pump was turned on at a debit rate of 160 ml/min, a vacuum was created in containers 2 and 3 and the sucrose from the container 1 was firstly diluted in the container 2, the content of which was further diluted in the container 3, the content of which being injected into the rotor (Figure 3A). When the container 1 was empty, the gradient was formed in the rotor and the centrifuge was started. Centrifugation was performed at 35000 rpm. After formation, successive fractions of 250 ml of the gradient were collected and analyzed for their sucrose content by refractometry (% Brix) in order to check the linearity of the gradient. Results are shown in Figure 3B.

### Results - Conclusions

Using the above method relying on the successive progressive dilution of a single stock solution of 55% sucrose prior to injection in a 8L rotor, a gradient was obtained which is substantially linear, *i.e.* linear over most of its volume. In particular, no significant plateau at each end of the gradient was observed (around 55% sucrose and 5% sucrose), as opposed to the two plateaux observed in Figure 2 at such sucrose percentages. The linear part of the gradient extended from about 1L to about 7L, *i.e.* the gradient was linear over 6L, that is over about 75% of its total volume. The inventors also checked the linearity of the gradient before centrifugation and they observed a similar curve as the one presented in Figure 3B.
**III.2** The same centrifuge and rotor as in Example II were used. 2 containers were used. The first one (container 1) contained 4L of PBS/Citrate 125mM buffer comprising 55% sucrose. The second one (container 2) contained 1L of PBS/Citrate 125mM buffer. The container 1 was connected to the container 2 that was connected through a pump to the bottom part of the K10/8L rotor that was filled up with PBS/Citrate 125mM buffer. Liquid in container 2 was mixed by a magnetic stirrer at a speed of 90 rpm. When the pump was turned on at a debit rate of 160 ml/min, a vacuum was created in container 2 and the sucrose from container 1 was diluted in container 2, the content of which was injected into the rotor (Figure 4A). When the container 1 was empty, the gradient was formed in the rotor and the centrifuge was started. Centrifugation was performed at 35000 rpm. After formation, successive fractions of 250 ml of the gradient were collected and analyzed for their sucrose content by refractometry (% Brix) in order to check the linearity of the gradient. Results are shown in Figure 4B.

### Results - Conclusions

Using the above method relying on the progressive dilution of a single stock solution of 55% sucrose prior to injection in a 8L rotor, a gradient was obtained which is linear over the first 5L of its volume, that is over about 62% of its total volume. A plateau at 5% sucrose was observed over the last 3L of the gradient, *i.e.* representing about 40% of its total volume. The inventors also checked the linearity of the gradient before centrifugation and they observed a similar curve as the one presented in Figure 4B.

### Example IV: Virus yield and purity

A linear 0-55% sucrose gradient was formed either in a K3/3.2L rotor as described in Example I or in a K10/8L rotor as described in Example III.1. Once the centrifuge reached a speed value of 35000 rpm, cell culture-derived whole influenza virus-containing fluid was loaded onto each rotor for virus purification. 17L of said fluid per L of rotor was loaded onto the 3.2L rotor and 35L of the same fluid per L of rotor was loaded onto the 8L rotor with a banding time of 1h. The purified whole virus was pooled from gradient fractions corresponding to a percentage of sucrose ranging from 23% to 50% (3.2L rotor) or 11% to 42% (8L rotor). These ranges have been determined on the basis of profiles from SDS-PAGE and from Western Blot analysis using anti-HA antibodies. HA (Haemagglutinin) protein content was assessed by SRD assay (as described in Example V) before and after the centrifugation, so that the percentage of "HA recovery" after the sucrose gradient centrifugation step could be calculated. "Purity" percentage represents the percentage of HA protein over the total proteins obtained at the end of the centrifugation. "Protein removal" percentage represents the elimination of total proteins further to the step of sucrose gradient centrifugation. The total protein concentration was assessed by Lowry method before and after the centrifugation, so that the "purity" percentage and the "Protein removal" percentage after the sucrose gradient centrifugation step could be calculated. Results are presented in Table 1.

**Table 1 - Virus yield and purity after sucrose gradient ultracentrifugation**

| | **3.2L rotor** | **8L rotor** |
|---|---|---|
| **HA recovery (%)** | 73 | 70 |
| **Purity (%)** | 21 | 24 |
| **Protein removal (%)** | 26 | 30 |

### Results - Conclusions

The method to create a pre-formed linear sucrose gradient in a high capacity rotor of 8L allows to obtain influenza virus (after purification by centrifugation) with a similar yield and a similar purity level as when using a smaller rotor of 3.2L. Such a result indicates that, with such a method, virus purification by density gradient centrifugation can be scaled up by reducing the number of equipments/runs required, with no impact on the product quality or quantity (virus yield and purity).

### Example V: SRD method used to measure HA content

Glass plates (12.4 - 10 cm) were coated with an agarose gel containing a concentration of anti-influenza HA serum that is recommended by NIBSC. After the gel has set, 72 sample wells (3 mm diameter) were punched into the agarose. 10 µl of appropriate dilutions of the reference and the sample were loaded in the wells. The plates were incubated for 24 hours at room temperature (20 to 25°C) in a moist chamber. After that, the plates were soaked overnight with NaCI solution and washed briefly in distilled water. The gel was then pressed and dried. When completely dry, the plates were stained on Coomassie Brillant Blue solution for 10 minutes and destained twice in a mixture of methanol and acetic acid until clearly defined stained zones become visible. After drying the plates, the diameter of the stained zones surrounding antigen wells was measured in two directions at right angles. Alternatively equipment to measure the surface can be used. Dose-response curves of antigen dilutions against the surface were constructed and the results were calculated according to standard slope-ratio assay methods (Finney, D.J. (1952). Statistical Methods in Biological Assay. London: Griffin, Quoted in: Wood, JM, et al (1977). J. Biol. Standard. 5, 237-247).

## Claims

1. A method for purifying a virus comprising at least the following steps:
a) obtaining a virus-containing fluid,
b) providing a pre-formed linear density gradient of a given range X%-Y% (v/w) (X being the lower limit and Y being the upper limit) in a centrifuge rotor having a capacity of at least 8L,
c) adding the fluid to the pre-formed linear density gradient,
d) centrifuging so as to separate the virus from contaminating impurities, and
e) collecting the fractions comprising the purified virus,
wherein the linear part of the gradient of the given range X%-Y% encompasses the density percentage corresponding to the percentage, in the gradient, at which the virus to be purified will migrate.

2. The method according to claim 1, wherein the separation is isopycnic.

3. The method according to claim 1 or claim 2, wherein the pre-formed linear density gradient of step b) is linear over at least 30%, at least 50%, at least 60%, at least 70%, at least 80%, at least 90% of its total volume or is linear over the entirety of its volume.

4. The method according to claim 1 or claim 2, wherein the pre-formed linear density gradient of step b) comprises plateaux at its ends whose added volume does not exceed 70%, 50%, 40%, 30%, 20%, 10%, or 5% of its total volume.

5. The method according to claims 1 to 4, wherein the pre-formed linear density gradient of step b) is formed by diluting a gradient-forming stock solution prior to the injection into the rotor.

6. The method according to claim 5, wherein the gradient-forming stock solution is a solution at Y% (w/v).

7. The method according to claims 5 and 6, wherein the gradient-forming stock solution is diluted at least once in a dilution solution prior to the injection into the rotor.

8. The method according to claims 5 and 6, wherein the gradient-forming stock solution is diluted in a first dilution solution which diluted stock solution is diluted in a second dilution solution prior to the injection into the rotor.

9. The method according to claims 7 and 8, wherein the dilution solution(s) contain no gradient medium.

10. The method according to claims 7 to 9, wherein the dilution solution has, or dilution solutions have, a volume of at least 10%, at least 15%, at least 20%, at least 25%, at least 30%, or at least 50% of the gradient-forming stock solution.

11. The method according to claims 7 to 9, wherein the ratio of the volume of the gradient-forming stock solution over the volume of the dilution solution(s) is least 2:1, at least 3:1, at least 4:1, at least 5:1 or at least 6:1.

12. The method according to claims 5 to 11, wherein the gradient-forming stock solution and the dilution solution(s) are connected with one another and connected to the rotor.

13. The method according to claims 5 to 12, wherein the dilution is a progressive dilution relying on a drip system.

14. The method according to claim 13, wherein the drip system is achieved by creating vacuum in the container(s) containing dilution solution(s)

15. The method according to claims 1 to 14, wherein the pre-formed linear density gradient of step b) is a sucrose gradient.

16. The method according to claims 1 to 15, wherein the pre-formed linear gradient of step b) ranges from 0 to 55%.

17. The method according to claims 1 to 16, wherein the virus is propagated on cell culture.

18. The method according to claim 27, wherein the virus-containing fluid of step a) is the cell culture medium collected after infection of cells with the virus and release of the virus into the cell culture medium.

19. The method according to claims 1 to 18, wherein the virus is influenza virus.

20. The method according to claim 17 to 19, wherein the virus is propagated on duck embryonic stem cells, such as EB66® cells.

21. A method for preparing a vaccine comprising at least the following steps:
A) obtaining a virus-containing fluid,
B) providing a pre-formed linear density gradient of a given range X%-Y% (v/w) (X being the lower limit and Y being the upper limit) in a centrifuge rotor having a capacity of at least 8L, wherein the linear part of the gradient of the given range X%-Y% encompasses the density percentage corresponding to the percentage, in the gradient, at which the virus will migrate,
C) adding the fluid to the pre-formed linear density gradient,
D) centrifuging so as to separate the virus from contaminating impurities, and
E) collecting the fractions comprising the purified virus,
F) formulating the purified virus into a vaccine.

## Patentansprüche

1. Verfahren zum Aufreinigen eines Virus, umfassend wenigstens die folgenden Schritte:
a) Erhalten einer Virus-enthaltenden Flüssigkeit,
b) Bereitstellen eines im Voraus gebildeten linearen Dichtegradienten eines bestimmten Bereichs X%-Y% (v/w) (wobei X die untere Begrenzung und Y die obere Begrenzung ist) in einem Zentrifugenrotor mit einer Kapazität von wenigstens 8L,
c) Hinzufügen der Flüssigkeit zum im Voraus gebildeten linearen Dichtegradienten,
d) Zentrifugieren, um das Virus von kontaminierenden Unreinheiten zu trennen, und
e) Sammeln der Fraktionen, welche das aufgereinigte Virus umfassen,
wobei der lineare Teil des Gradienten des bestimmten Bereichs X%-Y% die Dichteprozentzahl einschließt, welche der Prozentzahl im Gradienten entspricht, zu welcher das aufzureinigende Virus migrieren wird.

2. Verfahren gemäß Anspruch 1, wobei die Trennung isopyknisch ist.

3. Verfahren gemäß Anspruch 1 oder Anspruch 2, wobei der im Voraus gebildete lineare Dichtegradient von Schritt b) über wenigstens 30%, wenigstens 50%, wenigstens 60%, wenigstens 70%, wenigstens 80%, wenigstens 90% seines Volumens linear ist oder über die Gesamtheit seines Volumens linear ist.

4. Verfahren gemäß Anspruch 1 oder Anspruch 2, wobei der im Voraus gebildete lineare Dichtegradient von Schritt b) Plateaus an seinen Enden umfasst, deren zusammengezähltes Volumen 70%, 50%, 40%, 30%, 20%, 10% oder 5% seines Gesamtvolumens nicht überschreitet.

5. Verfahren gemäß Ansprüchen 1 bis 4, wobei der im Voraus gebildete lineare Dichtegradient von Schritt b) durch Verdünnen einer Gradient-bildenden Stammlösung vor der Injektion in den Rotor gebildet wird.

6. Verfahren gemäß Anspruch 5, wobei die Gradient-bildende Stammlösung eine Lösung bei Y% (w/v) ist.

7. Verfahren gemäß Ansprüchen 5 und 6, wobei die Gradient-bildende Stammlösung vor der Injektion in den Rotor wenigstens einmal in einer Verdünnungslösung verdünnt wird.

8. Verfahren gemäß Ansprüchen 5 und 6, wobei die Gradient-bildende Stammlösung vor der Injektion in den Rotor in einer ersten Verdünnungslösung verdünnt wird, welche verdünnte Stammlösung in einer zweiten Verdünnungslösung verdünnt wird.

9. Verfahren gemäß Ansprüchen 7 und 8, wobei die Verdünnungslösung(en) kein Gradientenmedium enthalten.

10. Verfahren gemäß Ansprüchen 7 bis 9, wobei die Verdünnungslösung(en) ein Volumen von wenigstens 10%, wenigstens 15%, wenigstens 20%, wenigstens 25%, wenigstens 30% oder wenigstens 50% der Gradient-bildenden Stammlösung hat/haben.

11. Verfahren gemäß Ansprüchen 7 bis 9, wobei das Verhältnis des Volumens der Gradient-bildenden Stammlösung gegenüber dem Volumen der Verdünnungslösung(en) wenigstens 2:1, wenigstens 3:1, wenigstens 4:1, wenigstens 5:1 oder wenigstens 6:1 ist.

12. Verfahren gemäß Ansprüchen 5 bis 11, wobei die Gradient-bildende Stammlösung und die Verdünnungslösung(en) miteinander verbunden und mit dem Rotor verbunden sind.

13. Verfahren gemäß Ansprüchen 5 bis 12, wobei die Verdünnung eine progressive Verdünnung ist, welche auf einem Tropfsystem beruht.

14. Verfahren gemäß Anspruch 13, wobei das Tropfsystem durch das Erzeugen eines Vakuums in dem Behälter/den Behältern, enthaltend die Verdünnungslösung(en), erreicht wird.

15. Verfahren gemäß Ansprüchen 1 bis 14, wobei der im Voraus gebildete lineare Dichtegradient von Schritt b) ein Saccharosegradient ist.

16. Verfahren gemäß Ansprüchen 1 bis 15, wobei der im Voraus gebildete lineare Gradient von Schritt b) von 0 bis 55% reicht.

17. Verfahren gemäß den Ansprüchen 1 bis 16, wobei das Virus auf einer Zellkultur propagiert wird.

18. Verfahren gemäß Anspruch 27, wobei die Virus-enthaltende Flüssigkeit von Schritt a) das Zellkulturmedium ist, welches nach dem Infizieren von Zellen mit dem Virus und der Freisetzung des Virus in das Zellkulturmedium gesammelt wurde.

19. Verfahren gemäß Ansprüchen 1 bis 18, wobei das Virus Influenza-Virus ist.

20. Verfahren gemäß Ansprüchen 17 bis 19, wobei das Virus auf embryonalen Stammzellen der Ente, wie beispielsweise EB66®-Zellen, propagiert wird.

21. Verfahren zum Herstellen eines Impfstoffes, umfassend wenigstens die folgenden Schritte:
A) Erhalten einer Virus-enthaltenden Flüssigkeit,
B) Bereitstellen eines im Voraus gebildeten linearen Dichtegradienten eines bestimmten Bereichs X%-Y% (v/w) (wobei X die untere Begrenzung und Y die obere Begrenzung ist) in einem Zentrifugenrotor mit einer Kapazität von wenigstens 8L, wobei der lineare Teil des Gradienten des bestimmten Bereichs X%-Y% die Dichteprozentzahl einschließt, welche der Prozentzahl im Gradienten entspricht, zu welcher das aufzureinigende Virus migrieren wird,
C) Hinzufügen der Flüssigkeit zum im Voraus gebildeten linearen Dichtegradienten,
D) Zentrifugieren, um das Virus von kontaminierenden Unreinheiten zu trennen, und
E) Sammeln der Fraktionen, welche das aufgereinigte Virus umfassen,
F) Formulieren des aufgereinigten Virus in einen Impfstoff.

## Revendications

1. Procédé de purification d'un virus comprenant au moins les étapes suivantes :
a) l'obtention d'un fluide contenant un virus,
b) la fourniture d'un gradient de densité linéaire préformé d'une plage X %-Y % donnée (v/p) (X étant la limite inférieure et Y étant la limite supérieure) dans un rotor de centrifugeuse ayant une capacité d'au moins 8 l,
c) l'ajout du fluide au gradient de densité linéaire préformé,
d) une centrifugation afin de séparer le virus à partir des impuretés contaminantes, et
e) la collecte des fractions comprenant le virus purifié,
dans lequel la partie linéaire du gradient de la plage X %-Y % donnée englobe le pourcentage de densité correspondant au pourcentage, dans le gradient, auquel le virus à purifier migrera.

2. Procédé selon la revendication 1, dans lequel la séparation est isopycnique.

3. Procédé selon la revendication 1 ou la revendication 2, dans lequel le gradient de densité linéaire préformé de l'étape b) est linéaire sur au moins 30 %, au moins 50 %, au moins 60 %, au moins 70 %, au moins 80 %, au moins 90 % de son volume total ou est linéaire sur la totalité de son volume.

4. Procédé selon la revendication 1 ou la revendication 2, dans lequel le gradient de densité linéaire préformé de l'étape b) comprend des plateaux à ses extrémités dont le volume ajouté ne dépasse pas 70 %, 50 %, 40 %, 30 %, 20 %, 10 %, ou 5 % de son volume total.

5. Procédé selon les revendications 1 à 4, dans lequel le gradient de densité linéaire préformé de l'étape b) est formé par la dilution d'une solution mère formant gradient avant l'injection dans le rotor.

6. Procédé selon la revendication 5, dans lequel la solution mère formant gradient est une solution à Y % (p/v).

7. Procédé selon les revendications 5 et 6, dans lequel la solution mère formant gradient est diluée au moins une fois dans une solution de dilution avant l'injection dans le rotor.

8. Procédé selon les revendications 5 et 6, dans lequel la solution mère formant gradient est diluée dans une première solution de dilution laquelle solution mère diluée est diluée dans une seconde solution de dilution avant l'injection dans le rotor.

9. Procédé selon les revendications 7 et 8, dans lequel la ou les solutions de dilution ne contiennent aucun milieu de gradient.

10. Procédé selon les revendications 7 à 9, dans lequel la solution de dilution a, ou les solutions de dilution ont, un volume d'au moins 10 %, d'au moins 15 %, d'au moins 20 %, d'au moins 25 %, d'au moins 30 %, ou d'au moins 50 % de la solution mère formant gradient.

11. Procédé selon les revendications 7 à 9, dans lequel le rapport du volume de la solution mère formant gradient sur le volume de la solution ou des solutions de dilution est au moins de 2:1, au moins de 3:1, au moins de 4:1, au moins de 5:1 ou moins de 6:1.

12. Procédé selon les revendications 5 à 11, dans lequel la solution mère formant gradient et la ou les solutions de dilution sont connectées les unes aux autres et connectées au rotor.

13. Procédé selon les revendications 5 à 12, dans lequel la dilution est une dilution progressive reposant sur un système de goutte-à-goutte.

14. Procédé selon la revendication 13, dans lequel le système de goutte-à-goutte est obtenu par la création d'un vide dans le ou les récipients contenant la ou les solutions de dilution.

15. Procédé selon les revendications 1 à 14, dans lequel le gradient de densité linéaire préformé de l'étape b) est un gradient de saccharose.

16. Procédé selon les revendications 1 à 15, dans lequel le gradient linéaire préformé de l'étape b) va de 0 à 55 %.

17. Procédé selon les revendications 1 à 16, dans lequel le virus est propagé sur une culture cellulaire.

18. Procédé selon la revendication 27, dans lequel le fluide contenant un virus de l'étape a) est le milieu de culture cellulaire collecté après l'infection des cellules avec le virus et la libération du virus dans le milieu de culture cellulaire.

19. Procédé selon les revendications 1 à 18, dans lequel le virus est un virus de la grippe.

20. Procédé selon les revendications 17 à 19, dans lequel le virus est propagé sur des cellules souches embryonnaires de canard, comme des cellules EB66®.

21. Procédé de préparation d'un vaccin comprenant au moins les étapes suivantes :
A) l'obtention d'un fluide contenant un virus,
B) la fourniture d'un gradient de densité linéaire préformé d'une plage X %-Y % donnée (v/p) (X étant la limite inférieure et Y étant la limite supérieure) dans un rotor de centrifugeuse ayant une capacité d'au moins 8 l, dans lequel la partie linéaire du gradient de la plage X %-Y % donnée englobe le pourcentage de densité correspondant au pourcentage, dans le gradient, auquel le virus migrera,
C) l'ajout du fluide au gradient de densité linéaire préformé,
D) une centrifugation afin de séparer le virus à partir des impuretés contaminantes, et
E) la collecte des fractions comprenant le virus purifié,
F) la formulation du virus purifié en un vaccin.
